(19) Europäisches Patentamt
European Patent Office
Office européen des brevets

(11) Veröffentlichungsnummer: **0 280 119**
**A1**

(12) **EUROPÄISCHE PATENTANMELDUNG**

(21) Anmeldenummer: 88101991.3

(22) Anmeldetag: 11.02.88

(51) Int. Cl.⁴: C07J 1/00 , C07J 31/00 , C07J 41/00 , A61K 31/565

(30) Priorität: 20.02.87 DE 3705990

(43) Veröffentlichungstag der Anmeldung: 31.08.88 Patentblatt 88/35

(84) Benannte Vertragsstaaten: AT BE CH DE ES FR GB GR IT LI LU NL SE

(71) Anmelder: SCHERING AKTIENGESELLSCHAFT
Berlin und Bergkamen
Müllerstrasse 170/178 Postfach 65 03 11
D-1000 Berlin 65(DE)

(72) Erfinder: Kerb, Ulrich, Dr.
Prinzregentenstrasse 7
D-1000 Berlin 31(DE)
Erfinder: Nishino, Yukishige, Dr.
Lanzendorfer Weg 14
D-1000 Berlin 22(DE)
Erfinder: Henderson, David, Dr.
Jahnstrasse 17
D-1000 Berlin 28(DE)

(54) **1-Methyl-15alpha-(1-Oxyalkyl)-androsta-1,4-dien-3,17-dione, Verfahren zu deren Herstellung und diese enthaltende pharmazeutische Präparate.**

(57) 1-Methyl-15α-(1-oxyl-alkyl-androsta-1,4-dien-3,17-dione der allgemeinen Formel I

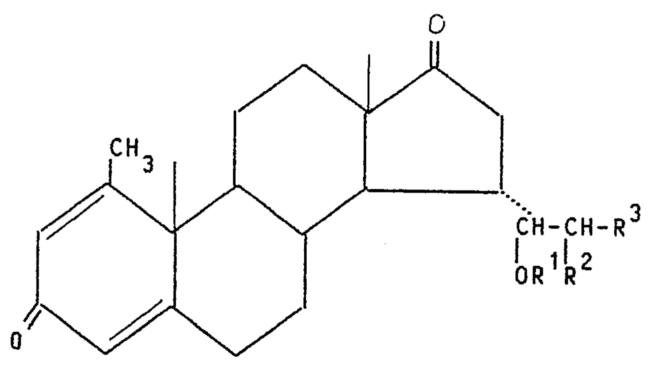

(I),

worin
$R^1$ ein Wasserstoffatom oder eine Acylgruppe mit 1 bis 10 Kohlenstoffatomen,
$R^2$ eine $OR^4$-, eine $NHR^5$-oder eine $S(O)_nR^6$-Gruppe,
$R^4$ eine gerad-oder verzweigtkettige Alkylgruppe mit 1 bis 10 Kohlenstoffatomen,
$R^5$ eine Acylgruppe mit 1 bis 10 Kohlenstoffatomen,
$R^6$ eine gerad-oder verzweigtkettige Alkylgruppe mit 1 bis 10 Kohlenstoffatomen oder eine Arylgruppe mit 6 bis 10 Kohlenstoffatomen,
$R^3$ ein Wasserstoffatom oder eine gerad-oder verzweigtkettige Alkylgruppe mit 1 bis 8 Kohlenstoffatomen und

EP 0 280 119 A1

Xerox Copy Centre

n 0, 1 oder 2
bedeuten,
sind als Inhibitoren der Östrogenbiosynthese zur Fertilitätskontrolle und zur Behandlung von Krankheiten geeignet, die durch Östrogene hervorgerufen werden.

# 1-METHYL-15α-(1-OXYALKYL)-ANDROSTA-1,4-DIEN-. 3,17-DIONE, VERFAHREN ZU DEREN HERSTELLUNG UND DIESE ENTHALTENDE PHARMAZEUTISCHE PRÄPARATE

Die Erfindung betrifft neue 1-Methyl-15α-(1-oxyalkyl)-androsta-1,4-dien-3,17-dione, Verfahren zu ihrer Herstellung, diese Verbindungen enthaltende pharmazeutische Präparate und ihre Verwendung zur Herstellung von Arzneimitteln.

Die erfindungsgemäßen Verbindungen werden durch die allgemeine Formel I beschrieben

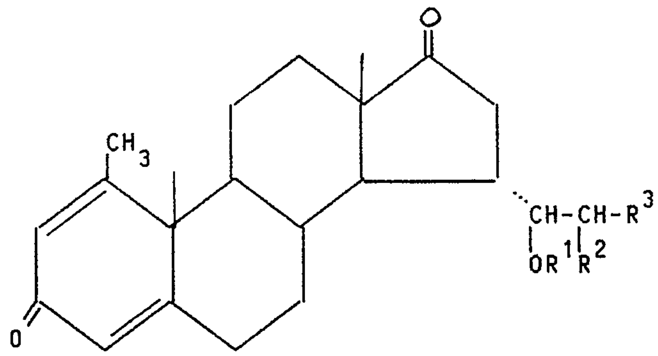

(I),

worin

$R^1$ ein Wasserstoffatom oder eine Acylgruppe mit 1 bis 10 Kohlenstoffatomen,

$R^2$ eine $OR^4$-, eine $NHR^5$-oder eine $S(O)_nR^6$-Gruppe,

$R^4$ eine gerad-oder verzweigtkettige Alkylgruppe mit 1 bis 10 Kohlenstoffatomen

$R^5$ eine Acylgruppe mit 1 bis 10 Kohlenstoffatomen,

$R^6$ eine gerad-oder verzweigtkettige Alkylgruppe mit 1 bis 10 Kohlenstoffatomen oder eine Arylgruppe mit 6 bis 10 Kohlenstoffatomen,

$R^3$ ein Wasserstoffatom oder eine gerad-oder verzweigtkettige Alkylgruppe mit 1 bis 8 Kohlenstoffatomen und

n 0, 1 oder 2

bedeuten.

Die für $R^1$ stehenden Acylgruppen enthalten 1 bis 10 Kohlenstoffatome. Besonders geeignet sind die Acetyl-, Propionyl-, Isopropinoyl und Butyrylgruppen.

Die für $R^3$ stehenden Alkylgruppen sind gerad-oder verzweigtkettig und enthalten 1 bis 8 Kohlenstoffatome, bevorzugt sind die Methyl-, Ethyl-, Propyl-, Butyl-, Isobutyl-und Pentylgruppe.

Die für $R^4$ stehenden gerad-oder verzweigtkettigen Alkylgruppen enthalten 1 bis 10 Kohlenstoffatome; vorzugsweise finden die Methyl-, Ethyl-, Propyl-und Isopropylgruppen Anwendung.

Die für $R^5$ stehenden Acylgruppen enthalten 1 bis 10 Kohlenstoffatome; bevorzugt sind die Acetyl-, Propionyl-und Isobutyrylgruppen.

Die für $R^6$ stehenden gerad-oder verzweigtkettigen Alkylgruppen mit 1 bis 10 Kohlenstoffatomen oder Arylgruppen mit 6 bis 10 Kohlenstoffatomen sind vorzugsweise die Methyl-, Ethyl-, Propyl-, Isopropyl-, Phenyl-, Toluyl-und Naphthylgruppen.

Es wurde nun gefunden, daß die neuen Verbindungen der allgemeinen Formel I als Aromatasehemmer die Östrogenbiosynthese hemmen.

So konnte zum Beispiel im sogenannten PMSG (Pregnant Mare's Serum Gonadotropin)-Test gezeigt werden, daß im Vergleich zu dem bekannten Aromatasehemmer 4-Hydroxy-4-androsten-3,17-dion die Serum-Östradiolkonzentration mit den erfindungsgemäßen Verbindungen stärker gesenkt wird.

Infantile weibliche Ratten reagieren auf PMSG-Behandlung mit einheitlich erhöhter Steroidsynthese. Die Aromataseaktivität kann durch die Beeinflussung der PMSG-stimulierten Östrogenbildung gemessen werden.

Im sogenannten PMSG-Test werden 21 Tage alte weibliche Ratten alle 2 Tage insgesamt 7 mal mit je 100 I.U. PMSG subkutan vorbehandelt. 1 Stunde vor und 8 Stunden nach der letzten PMSG-Applikation $(d_{12})$ erhalten die Tiere die Testsubstanz in 0,1 ml Benzylbenzoat/Rizinusöl (1 + 9) durch subkutane Injektion. Die Kontrolltiere erhalten nur das Vehikel. 24 Stunden nach der letzten PMSG-Applikation werden die Tiere getötet. Im Serum wird Östradiol radioimmunologisch geprüft. Für jede Gruppe von 10 Tieren wird der Mittelwert der Östradiolkonzentration in nMol/l mit der Standardabweichung errechnet. Die Signifikanzen der

Unterschiede zur Kontrollgruppe werden durch die Varianzanalyse geprüft.

Zur Ermittlung der relativen Wirkungsstärke der zu testenden Substanz im Vergleich zu der Standardsubstanz wird die Regressions-und Kovarianzanalyse durchgeführt. Ferner wird die prozentuale Hemmung gegen die PMSG-Kontrolle berechnet.

Am Beispiel der erfindungsgemäßen Verbindung 15α[(1R)-Acetoxy-2-methoxyethyl]-1-methyl-androsta-1,4-dien-3,17-dion (B) wird gezeigt, daß die erfindungsgemäßen Verbindungen die Östradiolkonzentration im Serum weit stärker senken als das bekannte 4-Hydroxy-4-androsten-3,17-dion (A). Während eine Hemmung der Östradiolkonzentration bei den erfindungsgemäßen Verbindungen schon mit 2x0,03 mg beobachtet wird, tritt eine solche Hemmung bei der Vergleichssubstanz erst mit 2x1,0 mg ein. Für die Verbindung B wird eine relative Wirkstärke von 6,3 gegenüber Verbindung A gefunden.

## TABELLE

Beeinflussung der Östradiol-Konzentration im peripheren Serum bei der PMSG-vorbehandelten Ratte

| | Dosis mg/Tier 2 x s.c. | n | Östradiol-Konzentration in nMol/l | Hemmung % | relative Wirkstärke |
|---|---|---|---|---|---|
| PMSG-Kontrolle | - | 10 | 6,21 ± 1,78 | - | - |
| A | 0,3 | 10 | 4,10 ± 1,05 | 34 | |
| | 1,0 | 10 | 2,38 ± 0,27 | 62 | 1,0 |
| | 3,0 | 10 | 0,81 ± 0,14 | 87 | |
| B | 0,03 | 10 | 2,73 ± 0,51 | 56 | |
| | 0,1 | 10 | 2,72 ± 0,51 | 56 | 6,3 |
| | 0,3 | 10 | 1,46 ± 0,25 | 76 | |
| | 1,0 | 10 | 0,62 ± 0,10 | 90 | |

n = Tierzahl pro Gruppe

Die Verbindungen sind Inhibitoren der Östrogenbiosynthese (Aromatasehemmer). Sie sind damit zur Behandlung von Krankheiten geeignet, die durch Östrogene bedingt oder von Östrogenen abhängig sind. So sind sie geeignet zur Behandlung von östrogeninduzierten oder - stimulierten Tumoren, wie zum Beispiel Mammakarzinom oder Prostatahyperplasie.

Die erfindungsgemäßen Verbindungen sind auch wertvoll zur Beeinflussung der Fertilität. So kann eine männliche Infertilität, die aus erhöhten Östrogenspiegeln resultiert, mit den neuen Wirkstoffen behoben werden.

Ferner können die Verbindungen bei Frauen im fortpflanzungsfähigen Alter als Antifertilitätsmittel verwendet werden, um Ovulation oder Implantation durch Östrogenentzug zu hemmen.
Wahrscheinlich sind Aromatasehemmer auch zur Behandlung des drohenden Herzinfarkts geeignet, da erhöhte Östrogenspiegel beim Mann einem Herzinfarkt vorausgehen kölnnen.

Die zu verabreichende Menge der Verbindung schwankt innerhalb eines weiten Bereichs und kann jede wirksame Menge abdecken. In Abhängigkeit des zu behandelnden Zustands und der ARt der Verabreichung kann die Menge der verabreichten Verbindung 0,01 - 100 mg/kg Körpergewicht, vorzugsweise 0,1 bis 20 mg/kg Körpergewicht, je Tag betragen.

Zur oralen Verabreichung kommen Kapseln, Pillen, Tabletten, Dragees usw. infrage. Die Dosierungseinheiten können neben dem Wirkstoff einen pharmazeutisch verträglichen Träger, wie zum Beispiel Stärke, Zucker, Sorbit, Gelantine, Gleitmittel, Kieselsäure, Talkum usw. enthalten. Die einzelnen Dosierungseinheiten für die orale Applikation können beispielsweise 10 bis 100 mg des Wirkstoffs (Aromatesinhibitors) enthalten.

Zur parenteralen Verabreichung können die Wirkstoffe in einem physiologisch verträglichen Verdünnungsmittel gelöst oder suspendiert sein. Als Verdünnungsmittel werden sehr häufig Öle mit oder ohne Zusatz eines Lösungsvermittlers, eines oberflächenaktiven Mittels, eines Suspendier-oder Emulgiermittels verwendet. Beispiele für verwendete Öle sind Olivenöl, Erdnußöl, Baumwollsamenöl, Sojabohnenöl, Rizinusöl und Sesamöl.

Die Verbindungen lassen sich auch in Form einer Depotinjektion oder eines Implantatpräparats anwenden, die so formuliert sein können, daß eine verzögerte Wirkstoff-Freigabe ermöglicht wird.

Implantate können als inerte Materialien zum Beispiel biologisch abbaubare Polymere enthalten oder synthetische Silikone, wie zum Beispiel Silikonkautschuk. Die Wirkstoffe können außerdem zur perkutanen Applikation zum Beispiel in ein Pflaster eingearbeitet werden.

Die Erfindung betrifft somit auch pharmazeutische Präparate und die Verwendung der neuen Verbindungen der allgemeinen Formel I zur Herstellung dieser Präparate zur Behandlung von östrogenenbedingten Krankheiten.

Die Erfindung betrifft auch ein Verfahren zur Herstellung von Verbindungen der allgemeinen Formel I, dadurch gekennzeichnet, daß man 17-Hydroxysteroide der allgemeinen Formel II

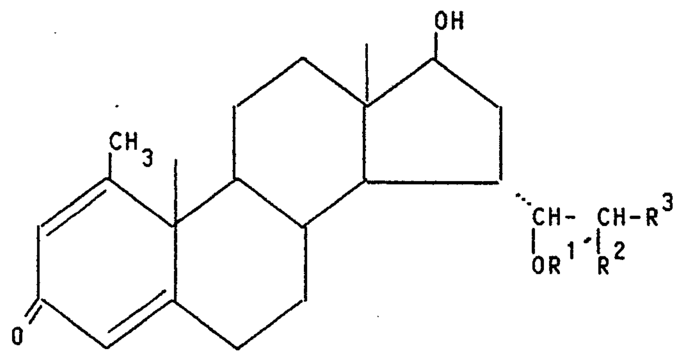

(II)

worin
$R^{1'}$ eine Hydroxyschutzgruppe oder eine Acylgruppe mit 1 bis 10 Kohlenstoffatomen,
$R^2$ eine $OR^4$-, eine $NHR^5$-oder eine $S(O)_nR^6$-Gruppe,
$R^4$ eine gerad-oder verzweigtkettige Alkylgruppe mit 1 bis 10 Kohlenstoffatomen,
$R^5$ eine Acylgruppe mit 1 bis 10 Kohlenstoffatomen,
$R^6$ eine gerad-oder verzweigtkettige Alkylgruppe mit 1 bis 10 Kohlenstoffatomen oder eine Arylgruppe mit 6 bis 10 Kohlenstoffatomen,
$R^3$ ein Wasserstoffatom oder eine gerad-oder verzweigtkettige Alkylgruppe mit 1 bis 8 Kohlenstoffatomen und
n 0, 1 oder 2
bedeuten,
in bekannter Weise oxidiert, gegebenenfalls die Hydroxyschutzgruppe abspaltet, gegebenenfalls die 15α-(1-Alkanoylgruppe) verseift, und den so erhaltenen Alkohol gegebenenfalls mit einer Carbonsäure mit 1 bis 10 Kohlenstoffatomen verestert.

Die Oxidation kann in an sich bekannter Weise zum Beispiel mit Chromsäurereagenzien (Jones-Reagenz oder Chromsäure-Pyridin) oder mit Pyridiniumdichromat oder -chlorochromat durchgeführt werden (J. Chem. Soc. 1953, 2555, Tetrahedron-Letters 1968, 3363, Tetrahedron-Letters 1979, 399).

Die in der allgemeinen Formel II für $R^{1'}$ stehenden Hydroxyschutzgruppen sind im sauren Milieu leicht abspaltbare Gruppen, wie zum Beispiel die Tetrahydropyranyl-, Methoxymethyl-oder Ethoxymethylgruppe.

Acylgruppen können mit einer anorganischen Base in alkoholischer Lösung verseift werden. Die sich gegebenenfalls anschließende Veresterung wird vorzugsweise mit dem entsprechenden Säureanhydrid oder -halogenid in Gegenwart von organischen Basen vorgenommen.

Das Ausgangsmaterial der allgemeinen Formel II wird durch nukleophile Öffnung des nach Vorschriften der deutschen Offenlegungsschrift P 35 39 244 hergestellten 15α-(1,2-Epoxyalkyl)-androstans der allgemeinen Formel III

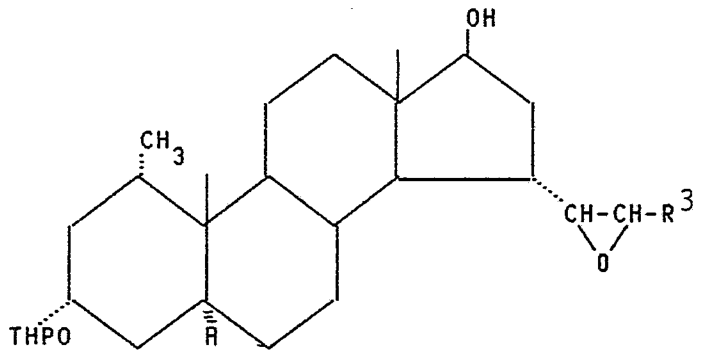

(III),

worin R die oben genannte Bedeutung hat,
sowie nach den ebenfalls dort beschriebenen Folgereaktionen (siehe auch experimenteller Teil) erhalten.

Die nukleophile Epoxidöffnung wird dabei nach literaturbekannten Verfahren (zum Beispiel Carl Djerassi, Steroid Reactions, San Francisco 1963, 615; Weygand/Hilgetag, Organisch-chemische Experimentierkunst, Leipzig 1970, 667) durchgeführt. So erfolgt die Einführung des $OR^4$-bzw. $SR^6$-Substituenten in die 15α-Alkylseitenkette durch Öffnen der Epoxide mit Alkalialkoholaten bzw. Alkalithioalkoholaten bei erhöhten Temperaturen. Die dabei erhaltenen Sulfide können durch geeignete Oxidationsmittel (z.B. Wasserstoffperoxid bzw. Persäuren) in die gewünschten Sulfoxide (n = 1) bzw. Sulfone (n = 2) überführt werden.

Verbindungen mit einem Aminosubstituenten in Position 2 der 15α-Alkylseitenkette werden durch meist mehrstündiges Erhitzen der Epoxide mit Alkaliaziden, wie zum Beispiel Natriumazid, in hochsiedenden, polaren organischen Lösungsmitteln, wie Ethylenglykol, Dimethylformamid, Alkoholen, zum Beispiel Methanol oder Ethanol, auf Temperaturen zwischen ca. 35 und 200°C sowie anschließende Reduktion der gebildeten Azide (z.B. Hydrierung mit Palladium-Calciumcarbonat als Katalysator) erhalten.

Die aminosubstituierten Verbindungen können auch durch Epoxidöffnung mit Aminen, z.B. in Ethylenglykol durch mehrstündiges Erhitzen auf ca. 100 bis 200 °C, hergestellt werden. Sie werden anschließend nach ebenfalls literaturbekannten Methoden acyliert.

**BEISPIEL 1**

a) 10,0 g 17β-Hydroxy-1α-methyl-15α-((1R)-oxiranyl)-3α-tetrahydropyranyloxy-5α-androstan (deutsche Offenlegungsschrift 35 39 244, Beispiel 3a) werden in 250 ml Methanol mit 13,5 g Natriummethylat 4 Stunden unter ArgonBegasung am Rückfluß erhitzt. Anschließend werden 14 ml Eisessig zugegeben, im Vakuum eingeengt und der Rückstand in Methylenchlorid gelöst. Nach Waschen mit Natriumhydrogencarbonat-Lösung und mit Wasser wird eingedampft. Man erhält so 11,3 g 17β-Hydroxy-15α-((1R)-hydroxy-2-methoxyethyl)-1α-methyl3α-tetrahydropyranyloxy-5α-androstan.

b) 11,3 g 17β-Hydroxy-15α-((1R)-hydroxy-2-methoxyethyl)-1α-methyl-3α-tetrahydropyranyloxy-5α-androstan werden in 40 ml Pyridin und 20 ml Acetanhydrid 16 Stunden bei 20°C gerührt. Es wird in Eiswasser gefällt, abgesaugt, gewaschen und getrocknet. Man erhält 12,87 g amorphes 17β-Acetoxy-15α-(-(1R)-acetoxy-2-methoxyethyl)-1α-methyl-3α-tetrahydropyranyloxy-5α-androstan, die in 200 ml 2-Propanol und 50 ml Wasser mit 2,5 g Oxalsäure 2 Stunden am Rückfluß erhitzt werden. Nach Eindampfen im Vakuum wird der Rückstand in Methylenchlorid gelöst, neutral gewaschen und das Lösungsmittel abdestilliert. 11,48 g der so erhaltenen 3α-Hydroxy-Verbindung werden in 100 ml Aceton mit 25 ml Jones-Reagenz 10 Minuten unter Wasserkühlung gerührt. Anschließend wird in Wasser gefällt und aufgearbeitet. Nach Umkristallisation aus Hexan/Aceton erhält man 7,5 g 17β-Acetoxy-15α-((1R)-acetoxy-2-methoxyethyl)-1α-methyl-5α-androstan-3-on.

Schmelzpunkt: 149-150°C

c) 7,17 g 17β-Acetoxy-15α-((1R)-acetoxy-2-methoxyethyl)-1α-methyl-5α-androstan-3-on werden in 95 ml Eisessig gelöst und unter Kühlung auf 15°C eine Lösung von 38.75 ml Brom in Eisessig (hergestellt aus 2,26 ml Brom in 50 ml Eisessig) innerhalb von 10 Minuten zugetropft und 10 Minuten nachgerührt. Nach Fällung in Eiswasser wird abgesaugt, neutral gewaschen und getrocknet. 10,9 g rohe 2,4-Dibromverbindung werden in 100 ml Dimethylformamid mit 10,0 g Lithiumcarbonat versetzt und 2 Stunden bei 140°C Badtemperatur gerührt. Nach Wasserfällung, Aufarbeitung und Umkristallisation aus Aceton/Hexan werden 4,28 g 17β-Acetoxy-15α-((1R)-acetoxy-2-methoxyethyl)-1-methyl-androsta-1,4-dien-3-on erhalten.
Schmelzpunkt: 187-188°C

d) 3,35 g 17β-Acetoxy-15α-((1R)-acetoxy-2-methoxyethyl)-1-methyl-androsta-1,4-dien-3-on werden in 60 ml Methanol mit 2 ml 3%iger Kaliumhydroxyd-Lösung in Methanol 4,5 Stunden bei Raumtemperatur gerührt. Nach Neutralisation mit Essigsäure, Aufarbeitung, Chromatographie an Silicagel und Umkristallisation aus Aceton/Hexan erhält man 1,3 g 17β-Hydroxy-15α-((1R)-acetoxy-2-methoxyethyl)-1-methyl-androsta-1,4-dien-3-on.
Schmelzpunkt: 203-204°C

e) 890 mg 17β-Hydroxy-15α-((1R)-acetoxy-2-methoxyethyl)-1-methyl-androsta-1,4-dien-3-on werden in 30 ml Aceton gelöst, mit 2,5 ml Jones-Reagenz versetzt und 45 Minuten bei Raumtemperatur gerührt. Nach Aufarbeitung, Chromatographie an Silicagel und Umkristallisation aus Aceton/Hexan erhält man 763 mg 15α-((1R)-Acetoxy-2-methoxyethyl)-1-methyl-androsta-1,4-dien-3,17-dion.
Schmelzpunkt: 184-185°C

**BEISPIEL 2**

Analog der im Beispiel 1 angegebenen Vorschrift wird 15α-((1R)-Acetoxy-2-ethoxyethyl)-1-methyl-androsta-1,4-dien-3,17-dion vom Schmelzpunkt 155-157°C erhalten.

**BEISPIEL 3**

a) 36,0 g 15α-Propenyl-17β-hydroxy-1α-methyl-3α-tetrahydropyranyloxy-5α-androstan (deutsche Offenlegungsschrift 35 39 244, Beispiel 15a)) werden in 900 ml Ethylenchlorid gelöst, mit 63,0 g p-Nitroperbenzoesäure versetzt und 1 Stunde bei 0-5°C gerührt. Nach Verdünnen mit Methylenchlorid wird mit 1 M Natriumhydroxidlösung und mit Wasser gewaschen und im Vakuum eingedampft. Durch Chromatographie an Silicagel und Kristallisation aus Ether-Pentan erhält man 27,7 g 17β-Hydroxy-15α-(1,2-epoxypropyl)-1α-methyl-3α-tetrahydropyranyloxy-5α-androstan vom Schmelzpunkt 172-174°C.

b) 26,5 g Epoxid werden in 550 ml Ethylenglykol mit 39,5 g Natriumazid 1 Stunde bei 180°C Badtemperatur unter Argon gerührt. Anschließend wird in Eiswasser gefällt, abgesaugt, mit Wasser gewaschen und getrocknet. Nach Chromatographie an Silicagel erhält man 20,6 g 17β-Hydroxy-15α-((1R)-hydroxy-(2R)-azidopropyl)-1α-methyl-3α-tetrahydropyranyloxy-5α-androstan vom Schmelzpunkt183-184°C und 4,5 g 17β-Hydroxy-15α-((1R)-hydroxy-(2S)-azidopropyl)-1α-methyl-3α-tetrahydropyranyloxy-5α-androstan vom Schmelzpunkt 141-143°C.

c) 20,6 g 17β-Hydroxy-15α-((1R)-hydroxy-(2R)-azidopropyl)-1α-methyl-3α-tetrahydropyranyloxy-5α-androstan werden in 500 ml Methanol mit 2,0 g 10%igem Palladium-Calciumcarbonat hydriert. Der Katalysator wird abfiltriert und das Methanol abdestilliert. 21,0 g rohe Aminoverbindung werden mit 80 ml Pyridin und 40 ml Acetanhydrid 16 Stunden bei Raumtemperatur stehengelassen. Nach Fällung in Wasser wird abgesaugt, mit Wasser gewaschen und getrocknet. Das Rohprodukt wird an Silicagel chromatographiert. Man erhält 24,4 g 17β-Acetoxy-15α-((1R)-acetoxy-(2R)-acetylaminopropyl)-1α-methyl-3α-tetrahydropyranyloxy-5α-androstan.

d) 24,4 g 17β-Acetoxy-15α-((1R)-acetoxy-(2R)-acetylaminopropyl)-1α-methyl-3α-tetrahydropyranyloxy-5α-androstan werden in 500 ml Methanol und 125 ml Wasser mit 6,5 g Oxalsäure 10 Minuten unter Rückfluß erhitzt. Nach der Aufarbeitung erhält man 20,3 g rohe 3α-Hydroxy-Verbindung, die in 250 ml Aceton mit 14 ml Jones-Reagenz oxidiert werden. Nach Aufarbeitung und Umkristallisation aus Methylenchlorid-Isopropylether werden 17,2 g 17β-Acetoxy-15α-((1R)-acetoxy-(2R)-acetylaminopropyl)-1α-methyl-5α-androstan-3-on erhalten.
Schmelzpunkt: 232-234°C

e) 16,2 g 17β-Acetoxy-15α-((1R)-acetoxy-(2R)-acetylaminopropyl)-1α-methyl-5α-androstan-3-on werden bromiert und dehydrobromiert, wie im Beispiel 1c beschrieben. Nach Chromatographie des Rohproduktes

und Umkristallisation aus Methylenchlorid-Isopropylether erhält man 7,35 g 17β-Acetoxy-15α-((1R)-acetoxy-(2R)-acetylaminopropyl)-1-methyl-androsta-1,4-dien-3-on.
Schmelzpunkt: 249-251 °C

f) 5,1 g 17β-Acetoxy-15α-((1R)-acetoxy-(2R)-acetylaminopropyl)-1-methyl-androsta-1,4-dien-3-on werden in 100 ml 0,1%iger Kaliumhydroxid-Lösung in Methanol 5 Stunden bei Raumtemperatur gerührt. Es wird mit Essigsäure neutralisiert und aufgearbeitet. Das Rohprodukt wird an Silicagel chromatographiert. Durch Eluieren mit tert.-Butylmethylether-Methanol (8:2) werden 1,88 g 17β-Acetoxy-15α-((1R)-hydroxy-(2R)-acetylaminopropyl)-1-methyl-androsta-1,4-dien-3-on erhalten, die in 6 ml Tetrahydrofuran und 3 ml Dihydropyran nach Zugabe von 0,03 g p-Toluolsulfonsäure 15 Stunden bei 20°C gerührt werden. Nach der Aufarbeitung erhält man 2,6 g 17β-Acetoxy-15α-((1R)-tetrahydropyranyloxy-(2R)-acetylaminopropyl)-1-methyl-androsta-1,4-dien-3-on, die in 50 ml 1%iger Kaliumhydroxid-Lösung 1 Stunde bei 20°C gerührt werden. Nach Aufarbeitung und Chromatographie isoliert man 2,05 g 17β-Hydroxy-15α-((1R)-tetrahydropyranyloxy-(2R)-acetylaminopropyl)-1-methyl-androsta-1,4-dien-3-on.

g) 1,8 g 17β-Hydroxy-15α-((1R)-tetrahydropyranyloxy-(2R)-acetylaminopropyl)-1-methyl-androsta-1,4-dien-3-on werden in 50 ml Methylenchlorid mit 5,2 g Pyridiniumdichromat 15 Stunden bei Raumtemperatur oxidiert. Es wird in Eiswasser gefällt, abgesaugt und getrocknet.1,45 g des so erhaltenen 17-Ketons werden in 24 ml Methanol und 6 ml Wasser mit 300 mg Oxalsäure 40 Minuten am Rückfluß erhitzt. Nach Aufarbeitung und Chromatographie isoliert man 1,1 g 15α-((1R)-Hydroxy-(2R)-acetylaminopropyl)-1-methyl-androsta-1,4-dien3,17-dion. 960 mg obiger Verbindung werden in 4 ml Pyridin und 2 ml Acetanhydrid umgesetzt und aufgearbeitet. Durch Umkristallisation aus Aceton-Hexan werden 945 mg 15α-((1R)-Acetoxy-(2R)-acetylaminopropyl)-1methyl-androsta-1,4-dien-3,17-dion erhalten.
Schmelzpunkt: 251-253°C

## BEISPIEL 4

4,5 g 17β-Hydroxy-15α-((1R)-hydroxy-(2S)-azidopropyl)-1α-methyl-3α-tetrahydropyranyloxy-5α-androstan (Beispiel 3b) werden umgesetzt, wie im Beispiel 3c-g beschrieben. Durch Umkristallisation aus Aceton-Ether werden 190 mg 15α-((1R)-Acetoxy-(2S)acetylaminopropyl)-1-methyl-androsta-1,4-dien-3,17-dion erhalten.
Schmelzpunkt: 179-180°C

## BEISPIEL 5

17β-Hydroxy-1α-methyl-15α-((1R)-oxiranyl)-3α-tetrahydropyranyloxy-5α-androstan (deutsche Offenlegungsschrift 35 39 244, Beispiel 3a) wird umgesetzt, wie in Beispiel 3 beschrieben. Man erhält so das amorphe 15α-((1R)-Acetoxy-2-acetylaminoethyl)1-methyl-androsta-1,4-dien-3,17-dion.

## BEISPIEL 6

a) 5 g 17β-Hydroxy-1α-methyl-15α-((1R)-oxiranyl)-3α-tetrahydropyranyloxy-5α-androstan (deutsche Offenlegungsschrift 35 39 244, Beispiel 3a) werden in 100 ml Ethylenglycol mit 7,0 g Natriumthiomethylat (hergestellt aus 4,8 g Methanthiol und 2,4 g Natriumhydrid) versetzt und 3 Stunden bis auf 100°C erhitzt. Dann wird wie im Beispiel 3b aufgearbeitet und chromatographiert. Man isoliert 2,1 g 17β-Hydroxy-15α-((1R)-hydroxy-(2R)-methylthioethyl)-1α-methyl-3α-tetrahydropyranyloxy-5α-androstan.

b) 2,0 g des nach 6a hergestellten Sulfids werden in 100 ml Acetonitril gelöst und mit einer Lösung von 5,2 g Natriummetaperiodat in 30 ml Wasser bei 50°C innerhalb von 2 Stunden versetzt. Anschließend wird mit Eiswasser verdünnt, die Kristalle abgesaugt, mit Wasser gewaschen und getrocknet. Nach Chromatographie an Kieselgel erhält man 1,06 g 2-(17β-Hydroxy-1α-methyl-3α-tetrahydropyranyloxy-5α-androstan-15α-yl)-2-hydroxyethyl-methyl-sulfoxid als Diastereomerenmischung.

d) Das nach b) hergestellte Sulfoxid wird wie in Beispiel 3c beschrieben acetyliert und analog den Vorschriften in Beispiel 3d bis 3g weiter umgesetzt. Man isoliert 105 mg 2-Acetoxy-2-(1-methyl-androst-1,4-dien-3,17-dion-15α-yl)-ethyl-methyl-sulfoxid.

**BEISPIEL 7**

a) 2,0 g des nach Beispiel 6b hergestellten Sulfids werden in 80 ml Dimethoxyethan gelöst und mit 5 ml 30%iger Peressigsäure unter Eiskühlung versetzt. Dann verdünnt man mit Eiswasser, filtriert die Kristalle ab, wäscht mit Wasser nach und trocknet. Die Kristalle werden an Kieselgel chromatographiert. Man isoliert 1,2 g 2-(17β-Hydroxy-1α-methyl-3α-tetrahydropyranyloxy-5α-androstan-15α-yl)-2-hydroxyethyl-methyl-sulfon.

b) Das nach a) hergestellte Sulfon wird wie in Beispiel 3c beschrieben acetyliert und analog den Vorschriften in Beispiel 3d bis 3g weiter umgesetzt. Man isoliert 120 mg 2-Acetoxy-2-(1-methyl-androst-1,4-dien-3,17-dion-15α-yl)-ethyl-methyl-sulfon.

## Ansprüche

1. 1-Methyl-15α-(1-oxyalkyl)-androsta-1,4-dien-3,17-dione der allgemeinen Formel I

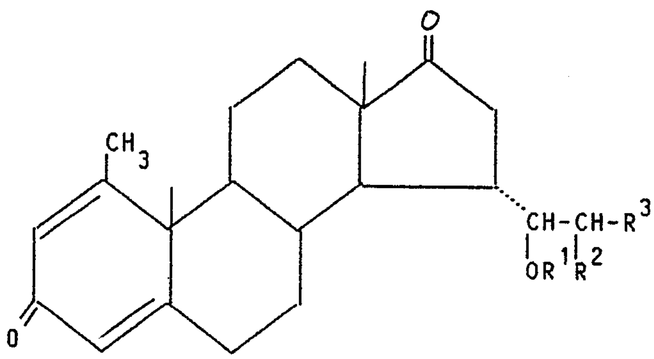

(I),

worin

$R^1$ ein Wasserstoffatom oder eine Acylgruppe mit 1 bis 10 Kohlenstoffatomen,

$R^2$ eine $OR^4$-, eine $NHR^5$-oder eine $S(O)_nR^6$-Gruppe,

$R^4$ eine gerad-oder verzweigtkettige Alkylgruppe mit 1 bis 10 Kohlenstoffatomen,

$R^5$ eine Acylgruppe mit 1 bis 10 Kohlenstoffatomen,

$R^6$ eine gerad-oder verzweigtkettige Alkylgruppe mit 1 bis 10 Kohlenstoffatomen oder eine Arylgruppe mit 6 bis 10 Kohlenstoffatomen,

$R^3$ ein Wasserstoffatom oder eine gerad-oder verzweigtkettige Alkylgruppe mit 1 bis 8 Kohlenstoffatomen und

n 0, 1 oder 2

bedeuten

2. 15α-((1R)-Acetoxy-2-methoxyethyl)-1-methyl-androsta-1,4-dien-3,17-dion.

3. 15α-((1R)-Acetoxy-2-ethoxyethyl)-1-methyl-androsta-1,4-dien-3,17-dion.

4. 15α-((1R)-Acetoxy-(2R)-acetylaminopropyl)-1-methyl-androsta-1,4-dien-3,17-dion.

5. 15α-((1R)-Acetoxy-(2S)-acetylaminopropyl)-1-methyl-androsta-1,4-dien-3,17 dion

6. 15α-((1R)-Acetoxy-2-acetylaminoethyl)-1-methyl-androsta-1,4-dien-3,17-dion.

7. 2-Acetoxy-2-(1-methyl-androsta-1,4-dien-3,17-dion-15α-yl)-ethyl-methylsulfoxid.

8. 2-Acetoxy-2-(1-methyl-androsta-1,4-dien-3,17-dion-15α-yl)-ethyl-methyl-sulfon.

9. Pharmazeutische Präparate, gekennzeichnet durch den Gehalt an mindestens einer Verbindung gemäß Ansprüchen 1 bis 8.

10. Verfahren zur Herstellung von 1-Methyl-15α-(1-oxyalkyl)-androsta-1,4-dien-3,17-dionen der allgemeinen Formel I

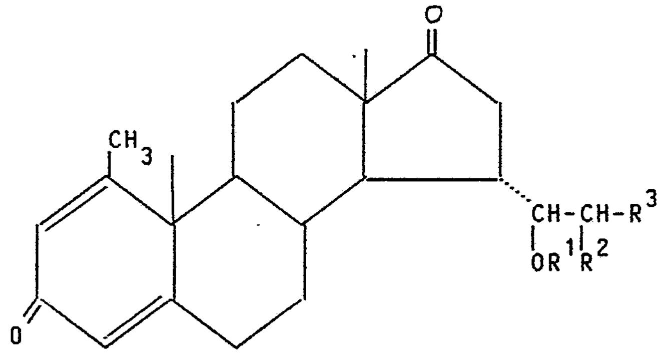

(I),

worin

$R^1$ ein Wasserstoffatom oder eine Acylgruppe mit 1 bis 10 Kohlenstoffatomen,

$R^2$ eine $OR^4$-, eine $NHR^5$-oder eine $S(O)_nR^6$-Gruppe,

$R^4$ eine gerad-oder verzweigtkettige Alkylgruppe mit 1 bis 10 Kohlenstoffatomen,

$R^5$ eine Acylgruppe mit 1 bis 10 Kohlenstoffatomen,

$R^6$ eine gerad-oder verzweigtkettige Alkylgruppe mit 1 bis 10 Kohlenstoffatomen oder eine Arylgruppe mit 6 bis 10 Kohlenstoffatomen,

$R^3$ ein Wasserstoffatom oder eine gerad-oder verzweigtkettige Alkylgruppe mit 1 bis 8 Kohlenstoffatomen und

n 0, 1 oder 2

bedeuten,

dadurch gekennzeichnet, daß man das 17-Hydroxysteroid der allgemeinen Formel II

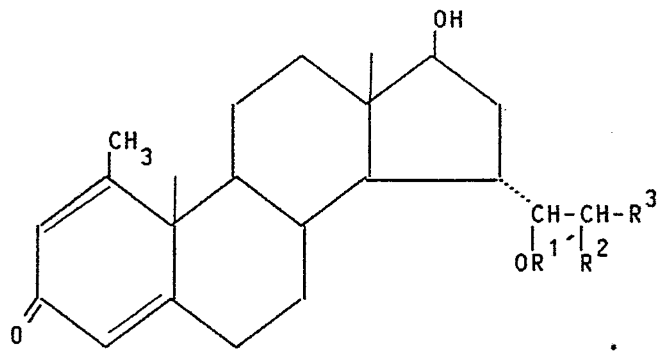

(II),

worin

$R^{1'}$ eine Hydroxyschutzgruppe oder eine Acylgruppe mit 1 bis 10 Kohlenstoffatomen,

$R^2$ eine $OR^4$-, eine $NHR^5$-oder eine $S(O)_nR^6$-Gruppe,

$R^4$ eine gerad-oder verzweigtkettige Alkylgruppe mit 1 bis 10 Kohlenstoffatomen,

$R^5$ eine Acylgruppe mit 1 bis 10 Kohlenstoffatomen,

$R^6$ eine gerad-oder verzweigtkettige Alkylgruppe mit 1 bis 10 Kohlenstoffatomen oder eine Arylgruppe mit 6 bis 10 Kohlenstoffatomen,

$R^3$ ein Wasserstoffatom oder eine gerad-oder verzweigtkettige Alkylgruppe mit 1 bis 8 Kohlenstoffatomen und

n 0, 1 oder 2

bedeuten,

in bekannter Weise oxidiert, gegebenenfalls die Hydroxyschutzgruppe abspaltet, gegebenenfalls die 15α-(1-Alkanoylgruppe) verseift und den so erhaltenen Alkohol gegebenenfalls mit einer Carbonsäure mit 1 bis 10 Kohlenstoffatomen verestert.

Europäisches Patentamt

# EUROPÄISCHER RECHERCHENBERICHT

Nummer der Anmeldung

EP 88 10 1991

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 129 500 (Patentansprüche 1,9; Seiten 2-9)<br>--- | 1,9 | C 07 J 1/00<br>C 07 J 31/00<br>C 07 J 41/00<br>A 61 K 31/565 |
| A | US-A-3 766 224 (R.V. COOMBS)<br>--- | | |
| D,P<br>X | EP-A-0 225 272 (SCHERING AG)<br>* Patentansprüche 1,8,9,10 *<br>----- | 1,9,10 | |
| | | | **RECHERCHIERTE SACHGEBIETE (Int. Cl.4)** |
| | | | C 07 J 1/00<br>C 07 J 31/00<br>C 07 J 41/00 |

Der vorliegende Recherchenbericht wurde für alle Patentansprüche erstellt

| Recherchenort | Abschlußdatum der Recherche | Prüfer |
|---|---|---|
| DEN HAAG | 25-05-1988 | HENRY J.C. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument